# EUROPÄISCHE PATENTANMELDUNG

(11) **EP 0 613 950 A1**
(43) Veröffentlichungstag der Anmeldung: **07.09.1994**
(21) Anmeldenummer: 94250015.8
(22) Anmeldetag: 27.01.1994
(51) Int. Cl.: C12P 1/00

(54) **Grenzflächenchemisch aktive Verbindungen aus nachwachsenden Rohstoffen**

(30) Priorität: 04.03.1993 DE 4307270
(71) Anmelder: AUF ANALYTIK UMWELTTECHNIK FORSCHUNG GmbH, D-12484 Berlin (DE)
(72) Erfinder: Olschewski, Max, D-12557 Berlin (DE); Olschewski, Brigitte, Dr., D-12557 Berlin (DE)
(74) Vertreter: Haupt, Wolfgang J.

(57) **Zusammenfassung**

Die Erfindung betrifft eine Mischung grenzflächenchemisch aktiver Verbindungen, die durch Reaktion von Hefen oder Bakterien mit natürlichen, pflanzlichen oder tierischen Fetten und Ölen erhalten werden, wobei die Reaktion im wäßrigen Medium und in Anwesenheit eines katalytisch wirksamen Mittels durchgeführt wird.

Diese Verbindungen sind als im wesentlichen nichtionische Tenside mit leichtem anionischen Anteil beispielsweise als Schäumungsmittel, Reinigungsmittel, Emulgatoren für die unterschiedlichsten organischen Stoffe in Wasser und für Wasser-in-Öl-Emulsionen sowie im weitesten Sinne als Netzmittel verwendbar.

## Beschreibung

Die Erfindung betrifft eine Mischung grenzflächenchemisch aktiver Verbindungen, die aus Hefen oder Bakterien einerseits und aus Fetten andererseits erhalten werden.
Diese Verbindungen sind als im wesentlichen nichtionische Tenside mit leichtem anionischen Anteil beispielsweise als Schäumungsmittel, Reinigungsmittel, Emulgatoren für die unterschiedlichsten organischen Stoffe in Wasser und für Wasser-in-Öl-Emulsionen sowie im weitesten Sinne als Netzmittel verwendbar.

Aufgrund günstiger Kosten-Nutzen-Relationen sind vor allem folgende Tenside in hoher Produktmenge auf den Markt gekommen:
Alkansulfonate, lineare Alkylbenzolsulfonate, Alkylethersulfate, Alkoholethoxylate, Alkylsulfate und Seife. Einige von ihnen, z.B. Alkylbenzolsulfate, sollten wegen ihren negativen ökologischen Eigenschaften substituiert werden.
Im Zeitalter einer zunehmenden Verknappung fossiler Rohstoffe und steigendem Umweltbewußtsein liegt der Schwerpunkt der Tensidchemie heute auf der Synthese von umweltverträglichen Tensiden auf Basis nachwachsender Rohstoffe [B.Fabry, Chemie in unserer Zeit 25 (1991) Nr.4]. Dabei zeichnen sich hinsichtlich eines guten Eigenschaftsbildes und günstigen ökologischen Vergleiches zwei wesentliche Tensidklassen ab, nämlich die Alkylpolyglucoside und die Eiweiß-Fettsäure-Kondensationsprodukte.
Zur Herstellung von Polyglucosiden werden entweder Saccharidhydroxylgruppen mit Fettalkoholen sauer veräthert (DE-40 06 192 und EP-448 799), Saccharide mit Fettsäuren oder Fettsäurechloriden verestert (EP-268 974) bzw. darüber hinaus noch oligoalkoxyliert (DE-41 03 681 und EP-405 967) oder auch Aldehydgruppen der Saccharide durch reduzierende Aminierung und anschließende Reaktion der Aminogruppen mit Säurechlorid zu Amiden umgesetzt (DE-35 38 451). Außerdem zeigen Zusätze solcher Polyglucoside zu Alkylethersulfat oder Alkansulfonat synergistische Effekte (DE-40 19 790). Alkylpolyglucoside besitzen interessante anwendungstechnische Eigenschaften wie synergistische Wechselwirkungen mit vielen wichtigen Tensiden, höhere Schaumaktivität als übliche nichtionische Tenside, verbesserte Hautverträglichkeit und gute biologische Abbaubarkeit.

Eine weitere Gruppe der milden Tenside mit guter Haut- und Schleimhautverträglichkeit sind die Eiweiß-Fettsäure-Kondensationsprodukte. Diese werden mit den normalen wasch- und reinigungsaktiven Mitteln kombiniert, um die Hautverträglichkeit der Alkylsulfate, α-Olefinsulfonate und Alkylbetaine zu verbessern. Dazu werden beispielsweise Eiweißhydrolysate der Molmasse 3000 bis 7000 mit Säurechlorid (EP-417 619 und EP-379 101) oder auch mit Fettsäureestern (DD-273 275) kondensiert. Die Eiweiß-Fettsäure-Kondensate werden immer in Kombination mit anderen Tensiden eingesetzt. Ihre Oberflächenspannung ist auch bei hohen Konzentrationen ≧ 30 mN/m. Die zu ihrer Herstellung dienenden Eiweißhydrolysate sind aus pflanzlichen und tierischen Proteinen erhältlich.
Die vorteilhaften Eigenschaften dieser Produkte bestehen in der fehlenden Toxizität, der reizhemmenden Wirkung in Kombination mit üblichen Tensiden und in einer guten Kompatibilität mit anionischen, nichtionischen sowie teilweise mit quaternären Ammoniumverbindungen. Sie besitzen selbst eine milde Reinigungswirkung.
Weiterhin werden Mono- und Diglyceride, d.h. Umesterungsprodukte von Fetten mit Fettsäuren, in der Lebensmittelchemie als Emulgatoren verwendet (DE-27 05 608, DE-19 37 433, DE-31 36 743, EP-0 305 901, EP-0 289 968).
Außerdem wurde durch die Anmelderin bereits vorgeschlagen, Hefe mit Säurehalogeniden, Alkylhalogeniden und Ethylenoxid zu grenzflächenchemisch aktiven Stoffen mit ungewöhnlichen Eigenschaften umzusetzen, jedoch ist hierfür die Verwendung eines Lösungsmittels erforderlich.

Die Erfindung bezweckt die Herstellung grenzflächenchemisch aktiver Verbindungen mit einer Oberflächenspannung der wäßrigen Lösung von ≦ 30 mN/m, die damit in besonderer Weise zur Benetzung von zahlreichen, unterschiedlichen polaren und unpolaren, aliphatischen und aromatischen Stoffen geeignet sind.

Es bestand dabei die Aufgabe, Hefen oder Bakterien, die zahlreiche und unterschiedliche reaktionsfähige Gruppen enthalten und die von Natur aus weder wasserlöslich sind noch nennenswerte grenzflächenchemische Eigenschaften aufweisen, in einfacher und ökonomisch günstiger Art und Weise durch chemische Umsetzung mit Fetten u.ä. in grenzflächenaktive Stoffe zu überführen.

Erfindungsgemäß wird aus Hefen oder Bakterien durch Umsetzung mit natürlichen, pflanzlichen und/oder tierischen Fetten und/oder Ölen in unterstöchiometrischen Mengen im wäßrigen Medium und in Anwesenheit von Alkalimetallhydroxiden als katalytisch wirksamen Mitteln eine Mischung grenzflächenchemisch aktiver Verbindungen hergestellt.

Geeignete Ausgangsprodukte sind z.B. Hefen wie Bäckerhefe, Brauhefe, Gärhefe in frischem Zustand wie auch als Abfallbiomasse oder Bakterien, z.B. in Form von Belebtschlamm. Ein möglicher, aber nicht notwendiger, partieller Aufschluß der Einsatzstoffe vor der Reaktion kann entweder im ameisensauren oder alkalischen Medium erfolgen, wobei enthaltene Ester und Amide ggfl. hydrolysiert werden.

Als besonders vorteilhaft ist die Tatsache hervorzuheben, daß die Reaktion überraschenderweise lediglich im wäßrigen Medium durchgeführt wird und demzufolge ohne irgendwelche Lösungsmittel auskommt.
Von gleichrangiger Bedeutung ist ebenfalls, daß die für die Reaktion eingesetzten Fette, auch Abfallfette, ohne jede Vorbehandlung verwendet werden können. Auch die Hefen oder Bakterien müssen im wesentlichen vorher nicht aufgeschlossen werden.

Die für die Umsetzung notwendigen katalytisch wirksamen Mittel sind Alkalimetallhydroxide.

Die erfindungsgemäß erhaltenen Produkte weisen eine ausgezeichnete biologische Abbaubarkeit auf.

Die Erfindung wird nachstehend ohne einschränkenden Charakter an Ausführungsbeispielen näher erläutert.

### BEISPIEL 1

400 g Brauhefe (25 %ig) mit einem Stickstoffgehalt von 7,9 % werden mit 160 g 40 %iger Natronlauge versetzt und 2 Stunden lang bei einer Temperatur von 90 °C gerührt. Dann gibt man 100 g Kokosfett hinzu und rührt das Ganze weitere 4 Stunden lang bei der gleichen Temperatur.
Nach dem Abkühlen erhält man ein etwa 40 %iges Batch von brauner Farbe und einem pH-Wert von 11.

### BEISPIEL 2

2000 g Gärhefe (30 %ig) mit einem Stickstoffgehalt von 8,7 % werden mit 800 g 40 %iger Natronlauge versetzt und 1 Stunde lang bei einer Temperatur von 80 °C gerührt. Dann gibt man 300 g Rindertalg hinzu und rührt das Ganze weitere 3 Stunden lang bei der gleichen Temperatur.
Nach dem Abkühlen erhält man ein etwa 43 %iges Batch von hellbrauner Farbe und einem pH-Wert von 10.

### BEISPIEL 3

500 g Belebtschlamm (22,5 %ig) mit einem Stickstoffgehalt von 7,8 % werden mit 150 g 45 %iger Natronlauge versetzt und 1 Stunde lang bei einer Temperatur von 95 °C gerührt. Dann gibt man 80 g Palmfett hinzu und rührt das Ganze weitere 4 Stunden lang bei der gleichen Temperatur.
Nach dem Abkühlen erhält man ein etwa 35 %iges Batch von anthrazitgrauer Farbe und einem pH-Wert von 10,5.

### BEISPIEL 4

400 g Bäckerhefe (25 %ig) mit einem Stickstoffgehalt von 6,7 % werden mit 100 g 40 %iger Natronlauge versetzt und 1 Stunde lang bei einer Temperatur von 85 °C gerührt. Dann gibt man 125 g Sojaöl hinzu und rührt das Ganze weitere 4 Stunden lang bei der gleichen Temperatur.
Nach dem Abkühlen erhält man eine etwa 45 %ige viskose Lösung von honiggelber Farbe und einem pH-Wert von 11.

### BEISPIEL 5

2000 g Gärhefe (30 %ig) mit einem Stickstoffgehalt von 8,7 % werden mit 600 g 40 %iger Natronlauge versetzt und 1 Stunde lang bei einer Temperatur von 80 °C gerührt. Dann gibt man 300 g Maisöl und 100 g Schweineschmalz hinzu und rührt das Ganze weitere 4 Stunden lang bei der gleichen Temperatur.
Nach dem Abkühlen erhält man ein etwa 40 %iges Batch von brauner Farbe und einem pH-Wert von 10,5.

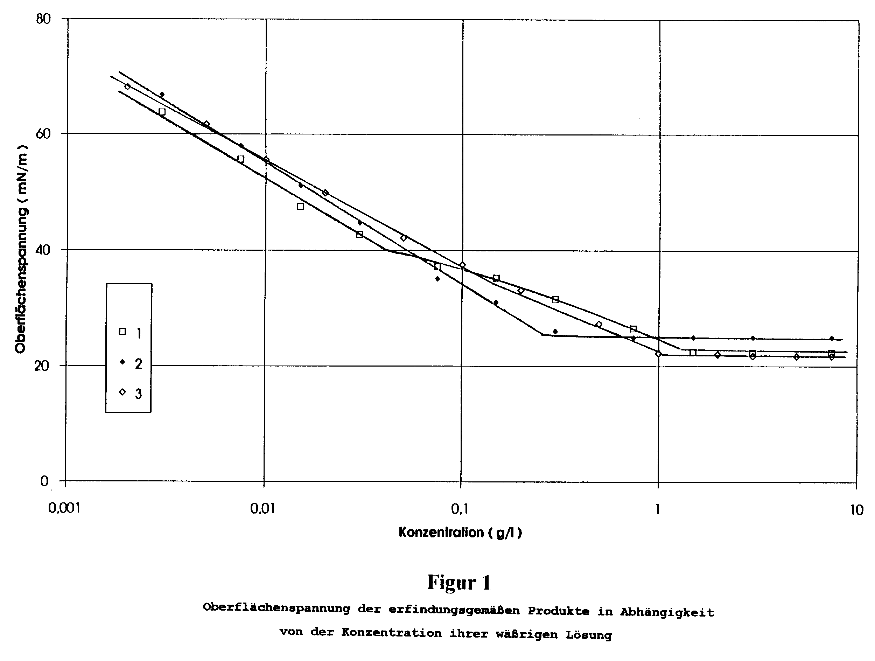

Die Tensid-Eigenschaften der erhaltenen Produkte sind in der nachfolgenden Tabelle I aufgeführt.
In der anliegenden Figur 1 ist anhand eines Diagrammes die Oberflächenspannung der Produkte in Abhängigleit von ihrer Konzentration in wäßriger Lösung dargestellt.

**TABELLE I**

| Produkt von Beispiel | Oberfl.-Spannung | | pH-Wert der Messung |
|---|---|---|---|
| | σ [mN/m] | CMC [g/l] | |
| 1 | 22,0 | 1,5·10^{o} | 7 |
| 2 | 24,9 | 3,5·10⁻¹ | 10 |
| 3 | 22,5 | 1,0·10^{o} | 10,5 |
| 4 | 27,7 | 2,0·10⁻¹ | 11 |
| 5 | 24,3 | 3,0·10⁻¹ | 7 |

## Patentansprüche

1. Mischung grenzflächenchemisch aktiver Verbindungen, dadurch gekennzeichnet, daß sie durch Reaktion von Hefen oder Bakterien mit natürlichen, pflanzlichen und/oder tierischen Fetten und/oder Ölen erhalten werden, wobei die Reaktion im wäßrigen Medium und in Anwesenheit von Alkalimetallhydroxiden als katalytisch wirksame Mittel durchgeführt wird und die genannten Fette und Öle in bezug auf die Gesamtheit der funktionellen Gruppen in den als Ausgangsprodukte verwendeten Hefen oder Bakterien in unterstöchiometrischen Mengen eingesetzt werden.
